# EUROPÄISCHE PATENTSCHRIFT

(11) **EP 0 563 614 B1**
(45) Veröffentlichungstag und Bekanntmachung des Hinweises auf die Patenterteilung: **21.01.1998**
(21) Anmeldenummer: 93103492.0
(22) Anmeldetag: 04.03.1993
(51) Int. Cl.: A61N 1/05, A61N 1/39

(54) **Defibrillierungselektrode**
Defibrillation electrode
Electrode de défibrillation

(30) Priorität: 16.03.1992 SE 9200803
(43) Veröffentlichungstag der Anmeldung: 06.10.1993
(73) Patentinhaber: Pacesetter AB, 171 95 Solna (SE)
(72) Erfinder: Hirschberg, Jakub, S-183 44 Täby (SE); Gilljam, Nina, S-133 44 Saltsjöbaden (SE); Neubauer, Heinz, S-175 70 Järfälla (SE); Bowald, Staffan, S-740 10 Almunge (SE)
(74) Vertreter: Lettström, Richard Wilhelm

(56) Entgegenhaltungen:
- EP-A- 0 009 732
- EP-A- 0 479 435
- US-A- 4 660 571
- US-A- 4 940 064
- US-A- 5 010 894

## Beschreibung

Die Erfindung betrifft eine Defibrillierungselektrode mit einem flexiblen Elektrodenkabel, das mindestens einen langgestreckten, elektrisch isolierten Leiter enthält, und mit einer am distalen Ende des Elektrodenkabels angebrachten Elektrodenanordnung, bestehend aus einer Anzahl langgestreckter, in nach aussen gerichteten Bögen vorgeformten flexiblen Leitern, deren eine Enden nebeneinander am distalen Ende des Elektrodenkabels und deren andere Enden nebeneinander an einem gemeinsamen Verbindungspunkt befestigt sind.

Eine Defibrillierungselektrode dieser Art ist durch die US-PS 5 010 894 bekannt. Diese Defibrillierungselektrode, die auch mit einer Herzschrittmacherelektrode kombiniert werden kann, ist nur für eine intrakardiale Stimulierung des Herzens vorgesehen. Bevor die Elektrode in eine Vene eingeführt wird, werden die vorgeformten Leiter der Elektrodenanordnung mit Hilfe eines Mandrins gestreckt, so dass diese dicht nebeneinander zu liegen kommen, wobei eine Einführung ins Herz ohne Schäden an den Wänden der Vene durchgeführt werden kann.

In der US-PS 4 030 509 ist ein sog. Patch-Modell einer Defibrillierungselektrode beschrieben. Eine solche Patch-Elektrode wird direkt am Myokardium oder am Herzsack angebracht. Diese Elektrode ist weder dafür geeignet noch dazu vorgesehen, ins Herz eingeführt zu werden.

EP-A-9.732 veröffentlicht einen Katheter für Herzschrittmacher mit einer Stimulationselektrode. Zur Erzielung einer sicheren und leichteren Positionierung der Stimulationselektrode und einer Sensorelektrode ist der Katheter in seinem vorderen Teil in einem dem Innendurchmesser des Ventrikels entsprechenden Abstand von der Spitze des Katheters und auf einer dem Durchmesser des Vorhofs entsprechenden Länge in zwei oder mehr Zweige aufgespalten, die relativ zur Katheterachse einen nach aussen gerichteten Bogen bilden.

Der Erfindung liegt die Aufgabe zugrunde, eine Defibrillierungselektrode der eingangs genannten Art zu schaffen, die sowohl für eine intrakardiale als auch für eine epikardiale Stimulierung des Herzens verwendet werden kann. Die Defibrillierungselektrode soll auch rasch ohne einen grösseren chirurgischen Eingriff in den Patienten eingeführt und am Epikardium oder am Herzsack plaziert werden können.

Diese Aufgabe ist erfindungsgemäss dadurch gelöst, dass die Leiter im Raum derart vorgeformt sind, dass die Befestigungsenden am gemeinsamen Verbindungspunkt gegenüber den Befestigungsenden am distalen Ende des Elektrodenkabels verdreht sind und dass Mittel vorhanden sind, mit denen der relative Abstand zwischen den Befestigungsenden der Leiter am gemeinsamen Verbindungspunkt und den Befestigungsenden der Leiter am distalen Ende des Elektrodenkabels variiert werden kann. Durch diesen Aufbau der Elektrodenanordnung können die Leiter gestreckt werden, so dass der gesamte Aussendurchmesser verringert wird. Dadurch kann die Elektrodenanordnung über eine Vene ins Herz eingeführt oder an der Aussenwand des Herzens oder am Herzsack appliziert werden. Durch die erwähnten Mittel kann auch der Abstand zwischen den Befestigungsenden derart verringert werden, dass die Leiter eine fast zweidimensionale Elektrodenkonfiguration aufweisen. Durch die beschriebene Verdrehung der Befestigungsenden der Leiter an dem gemeinsamen Verbindungspunkt gegenüber den Befestigungsenden am distalen Ende des Elektrodenkabels bilden dabei die Leiter Konturen von Blumenblättern, die sich überlappen. Auf diese Weise wird, abhängig von der Länge der Leiter, eine grosse bzw. kleine Patch-Elektrode erhalten. Die Dichte der Patch-Elektrode ist von der Anzahl der Leiter an der Elektrodenanordnung abhängig. Dadurch, dass der Aussendurchmesser der Defibrillierungselektrode bei einer Einführung ins Herz klein ist, ist auch der chirurgische Eingriff verhältnismässig klein. Die Defibrillierungselektrode kann vorzugsweise in der vena cava superior oder inferior appliziert und arretiert werden, in dem, wie beschrieben, die Leiter gegen die Venenwände gespannt werden.

In einer vorteilhaften Ausbildung der Erfindung wird vorgeschlagen, dass das Mittel ein Draht ist, der im Elektrodenkabel verschiebbar angeordnet ist und dessen distales Ende am Verbindungspunkt befestigt ist und dessen proximales Ende ausserhalb des proximalen Endes des Elektrodenkabels liegt.

Dadurch, dass ein sehr dünner und flexibler Draht verwendet werden kann, wird die Flexibilität des Elektrodenkabels nicht beeinflusst.

Als Alternative kann das Mittel ein Mandrin sein, der im Elektrodenkabel verschiebbar angeordnet ist und dessen distales Ende am Verbindungspunkt befestigt ist und dessen proximales Ende ausserhalb des proximalen Endes des Elektrodenkabels liegt. Durch den Mandrin können im Gegensatz zum Draht die Leiter gestreckt werden, wodurch der Aussendurchmesser der Elektrodenanordnung derart verringert wird, dass das Elektrodenkabel durch eine Vene geführt werden kann.

In einer vorteilhaften Weiterbildung der Erfindung wird vorgeschlagen, dass die Befestigungsenden der Leiter am gemeinsamen Verbindungspunkt gegenüber den Befestigungsenden am distalen Ende des Elektrodenkabels um einen Winkel von zwischen 120° und 240°, vorzugsweise 180° verdreht sind. Dieser Winkel muss in der Weise angepasst sein, dass sich die Leiter bei der beschriebenen Verringerung des Abstandes zwischen den Befestigungsenden etwa senkrecht zur Längsrichtung des Elektrodenkabels legen und teilweise überlappen.

Eine günstige Ausgestaltung der Erfindung ergibt sich, wenn der Verbindungspunkt ein Verbindungselement ist. Dieses Element kann eine Platte sein, auf der z.B. Befestigungsmittel angebracht werden können. Bei der Verwendung der Defibrillierungselektrode als eine Patch-Elektrode kann auf dem Verbindungselement eine schraubenförmige Spitze angebracht sein, die mittels eines Mandrins in den Herzmuskel eingeschraubt werden kann.

In einer weiteren Ausführungsform der Erfindung können die Leiter teilweise isoliert sein. Solche Isolierungen sind von Vorteil, wenn die Defibrillierungselektrode als eine intrakardiale Elektrode verwendet wird und wenn die Leiter zwischen den Herzwänden im Ventrikel gespannt werden. Die Teile der Leiter, die gegen die Herzwand anliegen, können in vorteilhafter Weise isoliert sein,was Brandwunden vermeidet.

Eine weitere günstige Ausgestaltung ergibt sich, wenn die Defibrillierungselektrode mit einer Herzschrittmacherelektrode kombiniert ist, deren Stimulationsoberfläche im Anschluss an den Verbindungspunkt der Leiter angebracht ist. Eine solche kombinierte Ausführungsform ist für eine intrakardiale Defibrillierungselektrode geeignet.

Im Hinblick auf eine günstige konstruktive Ausgestaltung dem Erfindung empfiehlt es sich, dass das Elektrodenkabel der Herzschrittmacherelektrode das Mittel ist, mit dem der relative Abstand zwischen den Befestigungsenden der Leiter am gemeinsamen Verbindungspunkt und den Befestigungsenden der Leiter am distalen Ende des Elektrodenkabels variiert werden kann.

Eine weitere Ausgestaltung der Erfindung ergibt sich, wenn die Elektrodenanordnung in einem Katheter verschiebbar angebracht ist, dessen Innendurchmesser geringfügig grösser ist als der Aussendurchmesser des Elektrodenkabels. Dadurch ist erreicht, dass die Defibrillierungselektrode ohne die Gefahr in den Patienten eingeführt werden kann, dass aufgrund nach aussen gerichteter Leiter Schäden an Venenwänden oder an anderen Geweben entstehen.

Die Erfindung ist nachfolgend anhand von mehreren, in den Zeichnungen dargestellten Ausführungsbeispielen näher erläutert. Es zeigen:
- FIG 1: eine Seitenansicht einer Defibrillierungselektrode mit einer Elektrodenanordnung nach der Erfindung,
- FIG 2: eine weitere Ausführungsform der Elektrodenan ordnung nach FIG 1 in einer perspektivischen Ansicht,
- FIG 3: eine Seitenansicht einer Elektrodenanordnung nach FIG 1 und 2 in Verbindung mit einem Introducer und
- FIG 4 und 5: weitere Ausführungsformen der Defibrillierungselektrode nach FIG 1 bis 3.

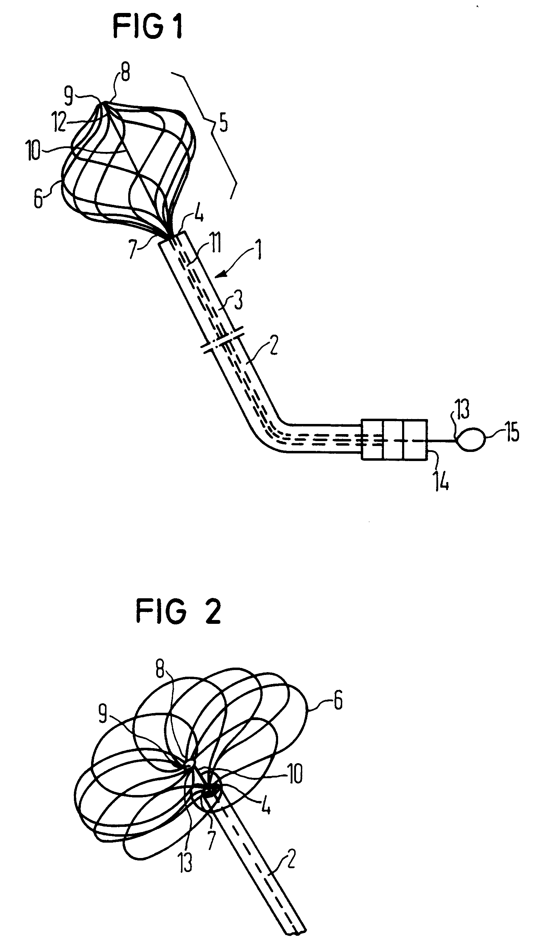

In der FIG 1 ist eine Defibrillierungselektrode 1 mit einem flexiblen Elektrodenkabel 2 abgebildet, das langgestreckte, elektrisch isolierte Leiter 3 enthält, die mit einer am distalen Ende 4 des Elektrodenkabels angebrachten Elektrodenanordnung 5 verbunden sind. Die Elektrodenanordnung 5 besteht aus einer Anzahl langgestreckter, in nach aussen gerichteten Bögenvorgeformter, flexibler Leiter 6, deren Enden 7 nebeneinander am distalen Ende 4 des Elektrodenkabels 2 und deren andere Enden 8 nebeneinander an einem gemeinsamen Verbindungspunkt 9 befestigt sind.Die Leiter 6 sind ausserdem im Raum derart vorgeformt, dass deren Befestigungsenden 8 am gemeinsamen Verbindungspunkt 9 gegenüber den Befestigungsenden 7 am distalen Ende 4 des Elektrodenkabels 2 verdreht sind. Der Drehwinkel beträgt zwischen 120° und 240°, vorzugsweise 180°. Die Defibrillierungselektrode 1 ist mit einem Draht 10 versehen, der am Elektrodenkabel 2 entlang verläuft, z.B. in einem Kanal 11, der durch die wendelförmige Konfiguration der Leiter 3 gebildet ist. Das distale Ende 12 des Drahts 10 ist am Verbindungspunkt 9 befestigt und das proximale Ende 13 liegt ausserhalb des proximalen Endes 14 des Elektrodenkabels 2. Das proximale Ende 13 des Drahts 10 kann vorzugsweise mit einer für den Benutzer behändigen Anordnung, wie z.B. einer Öse 15 versehen sein. Wenn der Benutzer an dem Draht 10 zieht, wird der relative Abstand zwischen den Befestigungsenden 8 der Leiter 6 am gemeinsamen Verbindungspunkt 9 und den Befestigungsenden 7 der Leiter 6 am distalen Ende 4 des Elektrodenkabels 2 variiert.

In der FIG 2 ist gezeigt, dass, wenn sich der Abstand zwischen den Befestigungsenden 7, 8 der Leiter 6 wesentlich verringert, diese eine fast zweidimensionale Elektrodenkonfiguration aufweisen, die die Konturen von sich überlappenden Blumenblättern bilden. Die in den FIG 1 und 2 beschriebene Defibrillierungselektrode kann vorzugsweise als Patch-Elektrode verwendet werden, die an der Aussenwand des Herzmuskels oder am Herzsack appliziert werden kann. Die Defibrillierungselektrode 1 kann auch bei einer intrakardialen Defibrillierung verwendet werden, bei der die Form der Elektrodenanordnung 5 mit Hilfe des Drahtes 10 gesteuert wird.

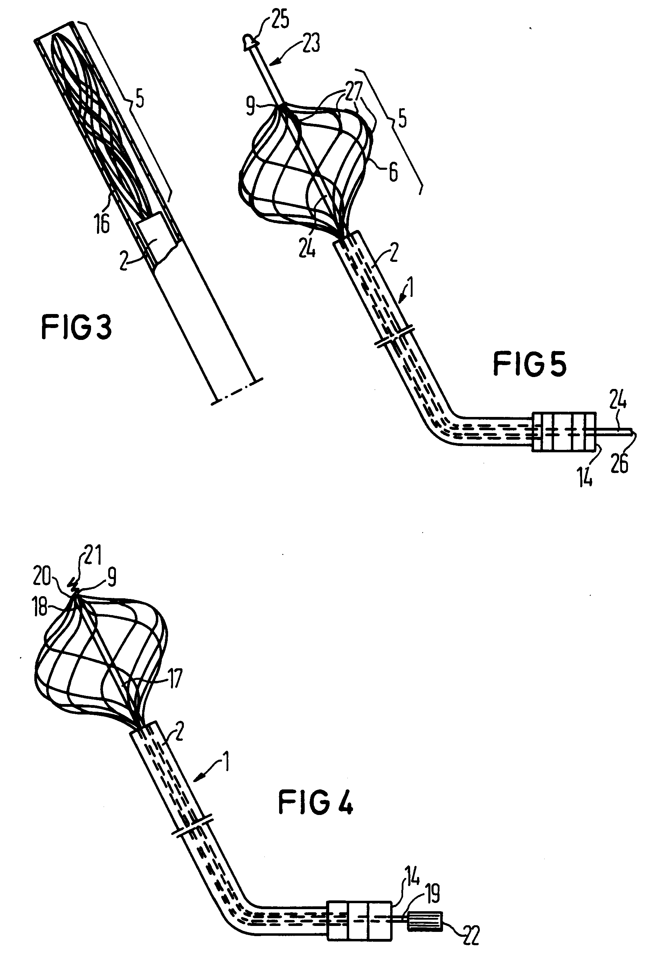

Bei einer Einführung der Defibrillierungselektrode 1 durch eine Vene ins Herz eines Patienten kann ein Introducer oder Katheter 16 verwendet werden, in dem die Elektrodenanordnung 5 und wenigstens ein Teil des Elektrodenkabels 2, wie es in der FIG 3 dargestellt, angebracht ist. Der Innendurchmesser des Katheters 16 ist dabei geringfügig grösser als der Aussendurchmesser des Elektrodenkabels 2, so dass die Elektrodenanordnung 5 ohne die Gefahr in den Ventrikel eingeführt werden kann, Schäden an Venenwänden oder am Herzen zu verursachen. Wenn die Defibrillierungselektrode 1 im Ventrikel appliziert ist, wird die Elektrodenanordnung 5 freigelegt, indem der Katheter 16 mittels bekannter und daher nicht dargestellter Mittel nach hinten verschoben wird oder durch Vorwärtsverschieben der Elektrodenanordnung 5 gegenüber dem Katheter 16, wobei die Elektrodenanordnung 5 eine, in der FIG 1 gezeigte, vorgeformte Konfiguration einnimmt. Eine Defibrillierungselektrode dieser Art kann auch in vena superior oder in vena inferior angebracht werden.

Die Defibrillierungselektrode 1 nach FIG 4 ist mit einem Mandrin 17 versehen, der den Draht 10 ersetzt. Der Mandrin 17 ist, wie der Draht 10, in dem Elektrodenkabel 2 verschiebbar angeordnet. Das distale Ende 18 des Mandrins 17 ist an dem Verbindungspunkt 9 drehbar befestigt und das proximale Ende 19 des Mandrins 17 liegt ausserhalb des proximalen Endes 14 des Elektrodenkabels 2. Der Verbindungspunkt 9 ist in diesem Ausführungsbeispiel ein Verbindungselement in Form einer Platte, die hier als Stütze für eine schraubenförmige Spitze 21, die an der Platte 20 drehbar befestigt ist, dient. Die schraubenförmige Spitze 21 ist mit dem Mandrin 17 verbunden. Durch Drehen eines Griffes 22, der am proximalen Ende 19 des Mandrins befestigt ist, in einer Richtung, wird die Spitze 21 entsprechend viel in dieselbe Richtung gedreht. Diese Defibrillierungselektrode eignet sich insbesondere als Patch-Elektrode, bei der die Spitze 21 als Befestigungsanordnung dient.

Der beschriebene Mandrin 17 kann auch in Verbindung mit der in den FIG 1 und 2 beschriebenen Defibrillatorelektrode 1 verwendet werden. Der Mandrin 17 kann im Unterschied zum Draht 10 die Elektrodenanordnung 5 derart strecken, dass sich die Leiter 6 ziemlich dicht an den Mandrin 17 und entlang diesem legen. Auf diese Weise ist eine Einführung der Defibrillierungselektrode 1 in einem Patienten ohne Hilfe eines Katheters 16 möglich.

Die in der FIG 5 gezeigte Defibrillierungselektrode 1 ist mit einer Herzschrittmacherelektrode 23 kombiniert, deren Elektrodenkabel 24 in dem Elektrodenkabel 2 der Defibrillierungselektrode 1 verschiebbar angeordnet ist. Das Elektrodenkabel 24 der Herzschrittmacherelektrode 23 ist am Verbindungspunkt 9 der Elektrodenanordnung 5 befestigt und ragt eine weitere Strecke über den Verbindungspunkt 9 hinaus. Das distale Ende des Elektrodenkabels 24 ist mit einem Elektrodenkopf 25 zum Übertragen von Stimulationsimpulsen ans Herz versehen. In diesem Ausführungsbeispiel dient das Elektrodenkabel 24 der Herzschrittmacherelektrode 23, dessen proximales Ende 26 ausserhalb des proximalen Endes 14 des Elektrodenkabels 2 liegt, als Mittel zum Variieren des relativen Abstandes zwischen den Befestigungsenden 7 und 8 der Leiter 6. Die hier beschriebene Defibrillierungselektrode ist speziell für eine intrakardiale Defibrillierung des Herzens vorgesehen, gleichzeitig damit, dass eine Herzstimulierung mit Hilfe eines Herzschrittmachers möglich ist. Diejenigen Teile 27 der Leiter 6, die gegen die Herzwände anliegen, sind isoliert, um Brennwunden zu vermeiden.

Der in Verbindung mit FIG 3 beschriebene Katheter 16 kann auch in Verbindung mit den in den FIG 4 und 5 gezeigten Defibrillierelektroden verwendet werden.

Die Länge der beschriebenen Elektrodenanordnung, d.h. die Länge der Leiter, kann variiert werden, insbesondere, wenn die Defibrillierungselektrode als Patch-Elektrode benutzt wird,um damit eine grosse oder eine kleine Stimulationsoberfläche zu erhalten. Die Zahl der Leiter kann auch variiert werden, um somit eine mehr oder weniger dichte Patch-Elektrode zu bekommen. Die Leiter, die einfache Drähte sein können, oder die geflochten oder wendelförmig sind, können auch so isoliert werden, dass verschiedene Formen und Grössen der Stimulationsoberflächen und eine gewünschte Anzahl Stimulationsoberflächen erhalten werden, wenn die Elektrodenanordnung in eine blumenblätterförmig aufgespannte Lage gebracht ist.

Die Defibrillierungselektrode nach der Erfindung kann auch ausser an den bereits beschriebenen Stellen an der Brustwand, subcutan, subpleural oder zwischen den Rippen und parallel mit diesen angebracht werden.

### Bezugszeichenliste

- 1: Defibrillierungselektrode
- 2, 24: Elektrodenkabel
- 3: Leiter
- 4: Distales Ende des Elektrodenkabels
- 5: Elektrodenanordnung
- 6: Leiter
- 7, 8: Leiterende, Befestigungsende
- 9: Verbindungspunkt
- 10: Draht
- 11: Kanal
- 12: Distales Ende des Drahtes
- 13: Proximales Ende des Drahtes
- 14: Proximales Ende des Elektrodenkabels
- 15: Öse
- 16: Katheter
- 17: Mandrin
- 18: Distales Ende des Mandrins
- 19: Proximales Ende des Mandrins
- 20: Verbindungselement, Platte
- 21: Schraubenförmige Spitze
- 22: Griff
- 23: Herzschrittmacherelektrode
- 25: Elektrodenkopf, Stimulationsoberfläche
- 26: Proximales Ende der Herzschrittmacherelektrode
- 27: Isoliertes Teil

## Patentansprüche

1. Defibrillierungselektrode mit einem flexiblen Elektrodenkabel (2), das mindestens einen langgestreckten elektrisch isolierten Leiter (3) enthält und mit einer am distalen Ende (4) des Elektrodenkabels (2) angebrachten Elektrodenanordnung (5), bestehend aus einer Anzahl langgestreckter, in nach aussen gerichteten Bögen vorgeformten flexiblen Leitern (6), deren eine Enden (7) nebeneinander am distalen Ende (4) des Elektrodenkabels und deren andere Enden (8) nebeneinander an einem gemeinsamen Verbindungspunkt (9) befestigt sind, **dadurch gekennzeichnet**, dass die Leiter (6) im Raum derart vorgeformt sind, dass die Befestigungsenden (8) am gemeinsamen Verbindungspunkt (9) gegenüber den Befestigungsenden (7) am distalen Ende (4) des Elektrodenkabels (2) verdreht sind, dass Mittel (10, 17, 24) vorhanden sind, mit denen der relative Abstand zwischen den Befestigungsenden (8) der Leiter (6) am gemeinsamen Verbindungspunkt (9) und den Befestigungsenden (7) der Leiter (6) am distalen Ende (4) des Elektrodenkabels (2) variiert werden kann, und dass die Leiter (6)
derart vorgeformt und an ihren Enden (7, 8) derart befestigt sind, dass, wenn der Abstand zwischen den gegenüberliegenden Enden (7, 8) wesentlich kleiner wird, die Leiter (6) eine fast zweidimensionale Elektrodenkonfiguration aufweisen,die die Konturen von sich überlappenden Blumenblättern bilden.

2. Defibrillierungselektrode nach Anspruch 1, **dadurch gekennzeichnet**, dass das Mittel (10) ein Draht ist, der im Elektrodenkabel (2) verschiebbar angeordnet ist und dessen distales Ende (12) am Verbindungspunkt (9) befestigt ist und dessen proximales Ende (13) ausserhalb des proximalen Endes (14) des Elektrodenkabels (2) liegt.

3. Defibrillierungselektrode nach Anspruch 1, **dadurch gekennzeichnet**, dass das Mittel (17) ein Mandrin ist, der im Elektrodenkabel (2) verschiebbar angeordnet ist und dessen distales Ende (18) am Verbindungspunkt (9) befestigt ist und dessen proximales Ende (19) ausserhalb des proximalen Endes (14) des Elektrodenkabels (2) liegt.

4. Defibrillierungselektrode nach Anspruch 1, **dadurch gekennzeichnet**, dass die Befestigungsenden (8) der Leiter (6) am gemeinsamen Verbindungspunkt (9) gegenüber den Befestigungsenden (7) am distalen Ende (4) des Elektrodenkabels (2) um einen Winkel von zwischen 120° und 240°, vorzugsweise 180° verdreht sind.

5. Defibrillierungselektrode nach einem der Ansprüche 1 bis 4, **dadurch gekennzeichnet**, dass der Verbindungspunkt (9) ein Verbindungselement (20) ist.

6. Defibrillierungselektrode nach einem der Ansprüche 1 bis 5, **dadurch gekennzeichnet**, dass die Leiter (6) teilweise isoliert sind.

7. Defibrillierungselektrode nach einem der Ansprüche 1 bis 6, **dadurch gekennzeichnet**, dass die Defibrillierungselektrode (1) mit einer Herzschrittmacherelektrode (23) kombiniert ist, deren Stimulationsoberfläche (25) im Anschluss an den Verbindungspunkt (9) der Leiter (6) angebracht ist.

8. Defibrillierungselektrode nach Anspruch 7, **dadurch gekennzeichnet**, dass das Elektrodenkabel (24) der Herzschrittmacherelektrode (23) das Mittel ist, mit dem der relative Abstand zwischen den Befestigungsenden (8) der Leiter am gemeinsamen Verbindungspunkt (9) und den Befestigungsenden (7) der Leiter (6) am distalen Ende (4) des Elektrodenkabels (2) variiert werden kann.

9. Defibrillierungselektrode nach einem der Ansprüche 1 bis 8, **dadurch gekennzeichnet**, dass die Elektrodenanordnung (5) in einen Katheter (16) verschiebbar angebracht ist, dessen Innendurchmesser geringfügig grösser ist als der Aussendurchmesser des Elektrodenkabels (2).

## Claims

1. Defibrillation electrode having a flexible electrode cable (2), which contains at least one elongated, electrically insulated conductor (3), and having an electrode arrangement (5) mounted on the distal end (4) of the electrode cable (2), said electrode arrangement consisting of a number of elongated flexible conductors (6), which are preformed in outwardly-directed curves and the ends (7) of which are fastened next to each other at the distal end (4) of the electrode cable and the other ends (8) of which are fastened next to each other at a common connection point (9), characterised in that the conductors (6) in the open are preformed in such a way that the fastening ends (8) at the common connection point (9) are twisted with respect to the fastening ends (7) at the distal end (4) of the electrode cable (2), in that there are means (10, 17, 24) with which the relative distance between the fastening ends (8) of the conductors (6) at the common connection point (9) and the fastening ends (7) of the conductors (6) at the distal end (4) of the electrode cable (2) can be varied, and in that the conductors (6) are preformed in such a way and are fastened at their ends (7, 8) in such a way that when the distance between the opposing ends (7, 8) becomes substantially smaller, the conductors (6) have an almost two-dimensional electrode configuration formed by the contours of overlapping flower petals.

2. Defibrillation electrode according to claim 1, characterised in that the means (10) is a wire, which is shiftably arranged in the electrode cable (2) and the distal end (12) of which is fastened to the connection point (9) and the proximal end (13) of which lies beyond the proximal end (14) of the electrode cable (2).

3. Defibrillation electrode according to claim 1, characterised in that the means (17) is a mandrin, which is shiftably arranged in the electrode cable (2) and the distal end (18) of which is fastened to the connection point (9) and the proximal end (19) of which lies beyond the proximal end (14) of the electrode cable (2).

4. Defibrillation electrode according to claim 1, characterised in that the fastening ends (8) of the conductors (6) at the common connection point (9) are twisted with respect to the fastening ends (7) at the distal end (4) of the electrode cable (2) by an angle of between 120° and 240°, preferably 180°.

5. Defibrillation electrode according to one of the claims 1 to 4, characterised in that the connection point (9) is a connecting element (20).

6. Defibrillation electrode according to one of the claims 1 to 5, characterised in that the conductors (6) are partially insulated.

7. Defibrillation electrode according to one of the claims 1 to 6, characterised in that the defibrillation electrode (1) is combined with a heart pacemaker electrode (23), the stimulation surface (25) of which is mounted so as to be joined to the connection point (9) of the conductors (6).

8. Defibrillation electrode according to claim 7, characterised in that the electrode cable (24) of the heart pacemaker electrode (23) is the means with which the relative distance between the fastening ends (8) of the conductors at the common connection point (9) and the fastening ends (7) of the conductors (6) at the distal end (4) of the electrode cable (2) can be varied.

9. Defibrillation electrode according to one of the claims 1 to 8, characterised in that the electrode arrangement (5) is shiftably mounted in a catheter (16), the inside diameter of which is slightly larger than the outside diameter of the electrode cable (2).

## Revendications

1. Electrode de défibrillation comportant un câble (2) d'électrode souple, qui comprend au moins un conducteur (3) oblong isolé électriquement, et comportant un dispositif (5) pour électrode, mis en place à l'extrémité distale (4) du câble (2) d'électrode, constitué d'un certain nombre de conducteurs (6) souples oblongs, dont des arcs sont préformés en étant dirigés vers l'extérieur, dont certaines (7) des extrémités sont fixées les unes à côté des autres à l'extrémité (4) distale du câble d'électrode et dont les autres extrémités (8) sont fixées les unes à côté des autres à un point (9) commun de liaison, caractérisée en ce que les conducteurs (6) sont préformés dans l'espace, de telle sorte que les extrémités (8) de fixation au point (9) commun de liaison soient tordues par rapport aux extrémités (7) de fixation à l'extrémité distale (4) du câble (2) d'électrode, il est prévu des moyens (10,17, 24), par lesquels l'écart relatif entre les extrémités (8) de fixation des conducteurs (6) au point (9) commun de liaison et les extrémités (7) de fixation des conducteurs (6) à l'extrémité (4) distale du câble (2) d'électrode peut être modifié, et les conducteurs (6) sont préformés et sont fixés à leurs extrémités (7,8), de telle sorte que, lorsque la distance entre les extrémités (7,8) opposées devient beaucoup plus petite, les conducteurs (6) qui forment les contours de pétales de fleur se chevauchant, aient une configuration d'électrodes presque bidimensionnelle.

2. Electrode de défibrillation suivant la revendication 1, caractérisée en ce que le moyen (10) est un fil métallique, qui est monté coulissant dans le câble (2) d'électrode, dont l'extrémité distale (12) est fixée au point (9) de liaison et dont l'extrémité proximale (13) se trouve à l'extérieur de l'extrémité proximale (14) du câble (2) d'électrode.

3. Electrode de défibrillation suivant la revendication 1, caractérisée en ce que le moyen (17) est un mandrin, qui est monté coulissant dans le câble (2) d'électrode, dont l'extrémité distale (18) est fixée au point (9) de liaison et dont l'extrémité proximale (19) se trouve à l'extérieur de l'extrémité proximale (14) du câble (2) d'électrode.

4. Electrode de défibrillation suivant la revendication 1, caractérisée en ce que les extrémités (8) de fixation des conducteurs (6) au point (9) commun de liaison sont tordues par rapport aux extrémités (7) de fixation à l'extrémité (4) distale du câble (2) d'électrode d'un angle compris entre 120° et 240°, de préférence de 180°.

5. Electrode de défibrillation suivant l'une des revendications 1 à 4, caractérisée en ce que le point (9) de liaison est un élément (20) de liaison.

6. Electrode de défibrillation suivant l'une des revendications 1 à 5, caractérisée en ce que les conducteurs (6) sont partiellement isolés.

7. Electrode de défibrillation suivant l'une des revendications 1 à 6 caractérisé en ce que l'électrode (1) de défibrillation est combinée avec une électrode (23) de stimulateur cardiaque, dont la surface (25) de stimulation est raccordée au point (9) de liaison des conducteurs (6).

8. Electrode de défibrillation suivant la revendication 7, caractérisée en ce que le câble (24) de l'électrode (23) du stimulateur cardiaque est le moyen, par lequel la distance relative entre les extrémités (8) de fixation des conducteurs au point (9) commun de liaison et les extrémités (7) de fixation des conducteurs (6) à l'extrémité distale (4) du câble (2) d'électrode peut être modifiée.

9. Electrode de défibrillation suivant la revendication 1 à 8, caractérisée en ce que le dispositif (5) pour électrode est monté coulissant dans un cathéter (16), dont le diamètre intérieur est légèrement supérieur au diamètre extérieur du câble (2) d'électrode.
